(19) Europäisches Patentamt
European Patent Office
Office européen des brevets

(11) Publication number: 0 314 199 B1

# (12) EUROPEAN PATENT SPECIFICATION

(45) Date of publication of patent specification: 18.09.91 Bulletin 91/38

(51) Int. Cl.$^5$: C07J 73/00, A61K 31/435

(21) Application number: 88119105.0

(22) Date of filing: 20.02.85

## (54) 17 Beta-substituted-4-aza-5 alpha-androstenones and their use as 5 alpha-reductase inhibitors.

The file contains technical information submitted after the application was filed and not included in this specification

(30) Priority: 27.02.84 US 584062
27.02.84 US 584061

(43) Date of publication of application: 03.05.89 Bulletin 89/18

(45) Publication of the grant of the patent: 18.09.91 Bulletin 91/38

(84) Designated Contracting States: AT BE CH DE FR GB IT LI LU NL SE

(56) References cited:
EP-A- 0 004 949

(56) References cited:
CHEMICAL ABSTRACTS, vol. 95, no. 13, 28th September 1981, page 99, no. 109055j, Columbus, Ohio, US; T. LIANG et al.: "Inhibition of 5alpha-reductase, receptor binding, and nuclear uptake of androgens in the prostate by a 4-methyl-4-aza-steroid", & J. BIOL. CHEM. 1981, 256(15), 7998-8005
JOURNAL OF ORGANIC CHEMISTRY, vol. 46, no. 7, July 1981, pages 1442-1446, American Chemical Society, Washington, D.C., US; T.G. BACK: "Oxidation of azasteroid lactams and alcohols with benzeneseleninic anhydride"

(60) Publication number of the earlier application in accordance with Art. 76 EPC: 0 155 096

(73) Proprietor: MERCK & CO. INC.
126, East Lincoln Avenue P.O. Box 2000
Rahway New Jersey 07065-0900 (US)

(72) Inventor: Rasmusson, Gary H.
155 Park Place
Watchung New Jersey 07060 (US)
Inventor: Reynolds, Glenn F.
252 Edgewood Avenue
Westfield New Jersey 07090 (US)

(74) Representative: Crampton, Keith John Allen et al
D. YOUNG & CO. 10 Staple Inn
London WC1V 7RD (GB)

EP 0 314 199 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention is concerned with 17β-substituted-4-aza-5α-androstenones, their preparation, and their use as testosterone-5α-reductase inhibitors.

It is well known that certain undesirable physiological manifestations, such as acne vulgaris, seborrhoea, female hirsutism, and male pattern baldness and benign prostatic hypertrophy, are the result of hyperandrogenic stimulation caused by an excessive accumulation of testosterone or similar androgenic hormones in the metabolic system. Early attempts to provide a chemotherapeutic agent to counter the undesirable results of hypernadrogenicity resulted in the discovery of several steroidal antiandrogens having undesirable hormonal activities of their own. The oestrogens, for example, not only counteract the effect of the androgens but have a feminizing effect as well. Non-steroidal antiandrogens have also been developed, for example, 4'-nitro-3'-trifluoromethylisobutyranilide [See Neri et al., Endo., Vol. 91, No. 1 (1972)]. However, these products, though devoid of hormonal effects, are peripherally active, competing with the natural androgens for receptor sites, and hence have a tendency to feminize a male host or the male foetus of a female host.

It more recently become known that the principal mediator of androgenic activity in some target organs is 5α-dihydrotestosterone, and that this is formed locally in the target organ by the action of testosterone-5α-reductase. It was therefore postulated and has been demonstrated that inhibitors of testosterone-5α-reductase serve to prevent or lessen symptoms of hyperandrogenic stimulation. Nayfe at al., [Steroids, 14, 269 (1969)] demonstrated that methyl 4-androsten-3-one-17β-carboxylate was a testosterone-5α-reductase inhibitor in vitro. Then Voigt and Hsia, Endocrinology, 92, 1216, (1973), Canadian Patent No. 970,692, demonstrated that the above ester and the parent free acid, 4-androsten-3-one-17β-carboxylic acid are both active inhibitors of testosterone-5α-reductase in vitro. They further demonstrated that topical application of either testosterone or 5α-dihydrotesterone caused enlargement of the female hamster flank organ, an androgen-dependent sebaceous structure. However, concomitant administration of 4-androsten-3-one-17β-carboxylic acid or its methyl ester inhibited the response elicited by testosterone but did not inhibit the response elicited by 5α-dihydrotestosterone. These results were interpreted as indicating that the compounds were antiandrogenic by virtue of their ability to inhibit testosterone-5α-reductase.

A number of 4-aza steroid compounds are known. See, for example, U.S. Patent Specifications Nos. US-A-2,227,876, 3,239,417, 3,264,301 and 3,285,918 ; French Patent Specification No. FR-A-1,465,544 ; Doorenbos and Solomons, J. Pharm. Sci. 62, 4, pp. 638-640 (1973) ; Doorenbos and Brown, J. Pharm. Sci., 60, 8, pp. 1234-1235 (1971) ; and Doorenbos and Kim, J. Pharm. Sci. 63, 4, pp. 620-622 (1974).

European Patent Specification EP-A-0004949 discloses inter alia that androstenone compounds of the formula :

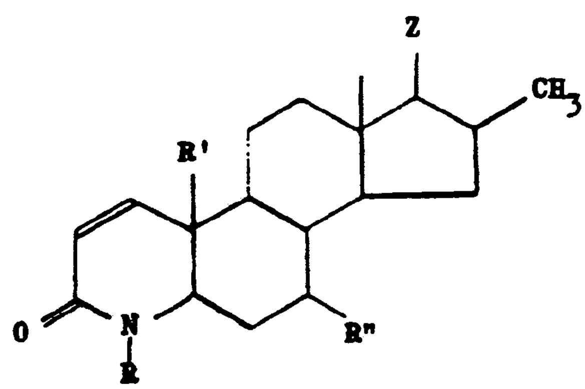

where R is hydrogen, methyl or ethyl ; $R^1$ is hydrogen or methyl ; R" is hydrogen or β-methyl ; and Z is CO-$R^8$, where $R^8$ is $C_{1-4}$ alkyl ; are active as testosterone 5α-reductase inhibitors.

In addition U.S. Patent Specifications Nos. US-A-4,377,584 and 4,220,775 of Rasmusson et al. describe a group of 4-aza-17β-substituted-5α-androstan-3-ones that are said to be useful in the treatment of hyperandrogenic conditions.

However, none of the documents mentioned above suggests that any of the novel 17β-acyl 4-aza-5α-androsten-1-en-3-ones of the present invention as defined below would be expected to have utility as highly potent testosterone-5α-reductase inhibitors.

The present invention provides novel 17β-acyl-4-aza-5α-androsten-1-en-3-one compounds of the formula:

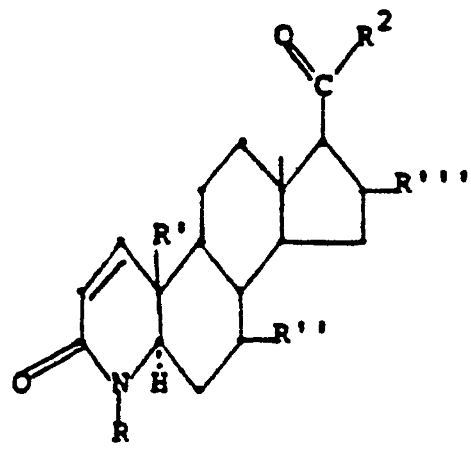

IA

in which R is hydrogen, methyl or ethyl ;
$R^2$ is a branched alkyl radical containing up to 12 carbon atoms ;
R′ is hydrogen or methyl ;
and each of R″ and R‴, which may be the same or different, is hydrogen, α-methyl or β-methyl.

Preferred compounds have the formula :

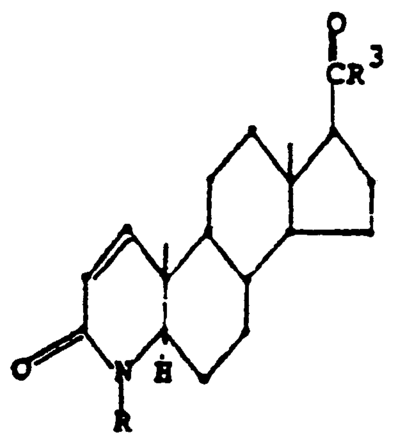

IIA

in which R is hydrogen, methyl or ethyl, and $R^3$ is branched chain alkyl of from 4-8 carbons.

Representative compounds of the present invention include the following :
17β-(t-butylcarbonyl)-4-aza-4-methyl-5α-androst-1-en-3-one ;
17β-(isobutylcarbonyl)-4-aza-4-methyl-5α-androst-1-en-3-one ;
17β-(isooctylcarbonyl)-4-aza-4-methyl-5α-androst-1-en-3-one ;
17β-(1,1-diethylbutyl-carbonyl)-4-aza-4-methyl-5α-androst-1-en-3-one ;
17β-(neopentylcarbamoyl)-4-aza-4-methyl-5α-androst-1-en-3-one ;
17β-(t-amylcarbonyl)-4-aza-4-methyl-5α-androst-1-en-3-one ;
17β-(t-hexylcarbonyl)-4-aza-4-methyl-5α-androst-1-en-3-one ;
and the corresponding compounds in which the 4-methyl substituent is replaced by a hydrogen atom or an ethyl radical.

The novel compounds of the present invention are prepared from the known steroid ester of the formula :

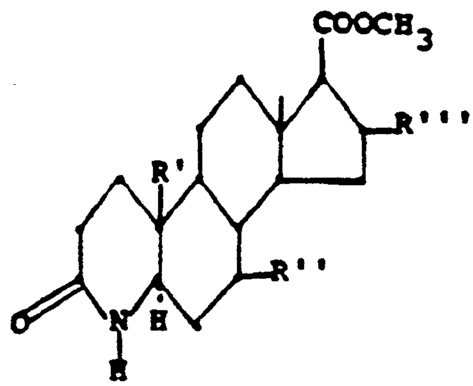

viz., 17β-(methoxycarbonyl)-4-aza-5α-androstan-3-one, or another 17β-alkoxycarbonyl-4-aza-5α-androstan-3-one compound, which may have the indicated substitution indicated by R′, R″ and R‴, by (A) dehydrogenating the starting material to produce the corresponding compound containing a double-bond in the 1,2-position of the A-ring, (B) converting the 17-substituent into a 17β-acyl substituent, and optionally (C) alkylating the A-ring nitrogen to introduce 4-methyl or 4-ethyl substituent into the A ring. In carrying out the process, it is essential that Stage (A) dehydrogenation of the 1,2-position of the steroid A ring be carried out using a 4-aza-5α-androstane-3-one-compound having no substituent other than hydrogen attached to the A-ring nitrogen. Stage (B) may consist of one or more chemical steps and if desired may take place before stage (A) or following stage (A) or stage (C).

In a practical method of carrying out this process, the products I are formed by (1) heating a 17β-alkoxycarbonyl-4-aza-5α-androstan-3-one compound III with a dehydrogenating agent such as benzeneselenic anhydride in refluxing chlorobenzene to form a 17β-alkoxycarbonyl-4-aza-5α-androst-1-en-3-one IV, (2) reacting the latter with sodium hydride under anhydrous conditions in a neutral solvent such as dimethylformamide, (3) contacting the resulting reaction mixture with an alkyl (e.g. methyl or ethyl) iodide to form the corresponding 17β-alkoxycarbonyl-4-alkyl-4-aza-5α-androst-1-en-3- one V, (4) subsequently hydrolysing the 17β-alkoxycarbonyl-4-alkyl-4-aza-5α-androst-1-en-3-one with a strong base such as aqueous methanolic potassium hydroxide at the reflux temperature, followed by acidification and isolation of the resulting steroidal acid 17β-carboxy-4-alkyl-4-aza-5α-androst-1-en-3-one VI, (5) converting the steroidal acid to its corresponding 2-pyridylthio ester by refluxing with triphenyl phosphine and 2,2′-dipyridyl disulphide in an inert solvent such as toluene and isolating the resulting product, viz. 17β-(2-pyridylthiocarbonyl)-4-alkyl-4-aza-5α-androst-1-en-3-one VII by chromatography on silica gel, and (6) reacting the pyridylthio ester with an $R^2$-Li or an $R^2MgX$ (X=Cl, Br) compound such as secbutylmagnesium chloride in tetrahydrofuran to form the desired product 17β-secbutylcarbonyl-4-alkyl-4-aza-5α-androst-1-en-3-one (VIIIA), which is isolated by chromatography on silica gel. When the previous reaction is carried out using an $R^2MgX$ or an $R^2$-Li compound other than secbutylmagnesium chloride, the corresponding 17β-acyl-4-alkyl-4-aza-5α-androst-1-en-3-one is prepared, where acyl is $R^2$ carbonyl.

The corresponding 17β-acyl-4-aza-5α-androst-1-en-3-one (XVA) is readily prepared from the 17β-(alkoxycarbonyl)-4-aza-5α-androsten-3-one (IV) by repeating the above series of reaction steps but omitting steps (2) and (3), i.e., treatment of the 4-aza-5α-androst-1-en-3-one with sodium hydride followed by methyl of ethyl iodide.

In accordance with a further alternative process of preparing the compounds of Formula IA having only hydrogen as the sole substituent on the ring A-nitrogen, the double bond in the A-ring is introduced as the last step of the process. Thus, a 17β-alkoxycarbonyl-4-aza-5α-androstan-3-one (III) is hydrolysed to the corresponding steroidal acid, 17β-carboxy-4-aza-5α-androstan-3-one (IX) which, in turn, is converted to the corresponding pyridylthio ester, 17β-(2-pyridylthiocarbonyl)-4-aza-5α-androstan-1-one (X) followed by treatment of the ester with an $R^2MgX$ or $R^2Li$ compound where $R^2$ is as defined hereinabove to form a 17β-acyl-4-aza-5α-androstan-3-one (XIA) which is dehydrogenated as previously described to produce compound XIV, 17β-acyl-4-aza-5α-androst-1-en-3-one.

The 16-methyl derivatives, i.e. compounds in which R‴ is methyl, are prepared from known 16-methyl-17-acyl-4-methyl-4-aza-5α-androstan-3-ones, e.g. 4,16β-dimethyl-17β-acetyl-4-aza-5α-androstan-3-one, by known dehydrogenation procedures for 4-methyl-4-aza compounds to produce the corresponding 4,16β-dimethyl-17β-acetyl-4-aza-5α-androst-1-en-3-one.

The above reactions are schematically represented in the following reaction scheme, in which X is 2-pyridylthio and the other variables are as defined above.

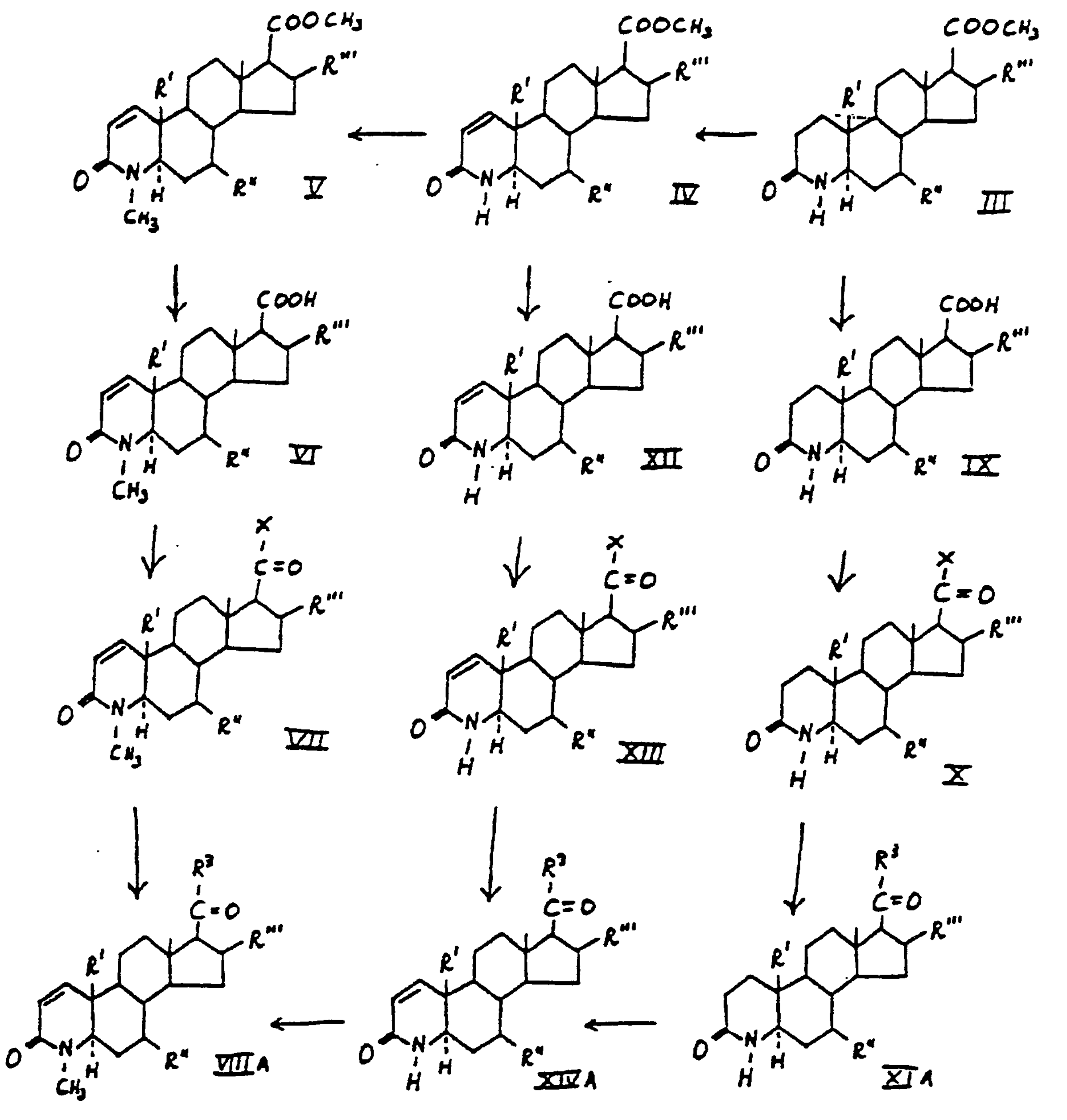

V IV III

VI XII IX

VII XIII X

VIIIA XIVA XIA

Compounds of the present invention, which may be prepared in accordance with the method described above, have been found to be potent antiandrogens by virtue of their ability to specifically inhibit testosterone-5α-reductase and are therefore suitable for treating the hyperandrogenic conditions of acne vulgaris, seborrhea and female hirsutism by topical administration, and for treating all of the above conditions, as well as benign prostatic hypertrophy, by oral or parenteral administration.

The present invention thus also provides oral, topical and parenteral pharmaceutical formulations for use in administering the active compounds of the present invention.

The compositions containing the compounds of the present invention as the active ingredient for use in the treatment of benign prostatic hypertrophy can be administered in a wide variety of therapeutic dosage forms in conventional vehicles for systemic administration, as, for example, by oral administration in the form of tablets, capsules, solutions or suspensions, or by intravenous injection. The daily dosage of the products may be

varied over a wide range varying from 50 to 2,000 mg. The compositions are preferably provided in the form of scored tablets containing 5, 10, 25, 50, 100, 150, 250 and 500 mg. of the active ingredient for the symptomatic adjustment of the dosage to the patient to be treated. An effective amount of the drug is ordinarily supplied at a dosage level of from 1 to 50 mg./kg. of body weight per day. Preferably the range is from 1 to 7 mg./kg. of body weight per day. These dosages are well below the toxic dose of the product. Capsules containing an active compound of this invention can be prepared by mixing it with lactose and magnesium stearate, calcium stearate. starch, talc, or other carriers, and placing the mixture in gelatin capsules. Tablets may be prepared by mixing the active ingredient with conventional tableting ingredients such as calcium phosphate, lactose, corn starch or magnesium stearate. The liquid forms in suitably flavoured suspending or dispersing agents such as the synthetic and natural gums, for example, tragacanth, acacia and methyl cellulose. Other suitable dispersing agents include glycerin. For parenteral administration, sterile suspensions and solutions are desired. Isotonic preparations, which usually contain suitable preservative, are prepared for intravenous administration.

For the treatment of acne vulgaris, seborrhoea and female hirsutism, the compounds of the present invention are administered in the form of pharmaceutical compositions comprising the active compound in combination with a pharmacologically acceptable topically administrable carrier. These topical pharmaceutical compositions may be in the form of a cream, ointment, gel or aerosol formulation suitable for application to the skin. They ordinarily include 0.1% to 15%, preferably about 5%, of the active compound, based on the total composition.

The method of preparing the novel 17β-acyl compounds of the present invention, already described above in general terms, is further illustrated by the following Examples.

EXAMPLE 1

Step A

Methyl 3-oxo-4-aza-5α-androst-1-ene-17β-carboxylate

A suspension of 83.7 g of methyl 3-oxo-4-aza-5α-androstane-17-carboxylate, obtained as in U.S. Patent Specification US-A-4 377 584, and 126.5 g of benzeneseleninic anhydride in 2.09 litres of chlorobenzene was heated at reflux for 2 hours. The reflux condenser was switched to a distillation head and the mixture was distilled slowly to remove water that had formed in the reaction (2 hours). The solution was evaporated to leave 198 g of wet residue. The residue as a solution in dichloromethane was washed with saturated aqueous $NaHCO_3$ solution and saturated NaCl solution, then dried and evaporated to leave 172.4 g. This material was chromatographed on 2.56 kg of silica gel eluting first with dichloromethane (5 litres) and then with 4 : 1 (v/v) dichloromethane-acetone. The desired product eluted after 8 litres and amounted to 53.4 g. It was rinsed with diethyl ether and dried to leave 49.5 g., m.p. 278-280°C.

Step B

Methyl 4-methyl-3-oxo-4-aza-5α-androst-1-ene-17β-carboxylate

A suspension of 25 g of the product of Step A and 2.25 g of sodium hydride in 500 ml of dry dimethylformamide was stirred under nitrogen for 15 minutes. 15 ml of methyl iodide was added dropwise and the mixture was stirred for 30 minutes at room temperature. An additional 5 ml of methyl iodide was added and the mixture was heated at 50°C for 2 hours. After cooling the mixture was diluted with water to 2 litres. The solid was separated after cooling and amounted to 25.4 g, m.p. 159-161°.

Step C

S-(2-Pyridyl)4-methyl-3-oxo-4-aza-5α-androst-1-ene-17β-thiocarboxylate

A suspension of 25 g of the product of Step B in 125 ml of methanol was treated with a solution of KOH (12.5 g) in 12.5 ml of water. After refluxing for 4 hours, the solution was acidified with 6N HCl and then was diluted with water. The crude acid (23.32 g) was separated and dried. It had m.p. 300°C.

The crude dry acid (23 g), triphenylphosphine (36.45 g) and 2,2'-dipyridyldisulphide (30.4 g) were suspended in 138 ml of toluene with stirring for 3 hours at room temperature. The reaction mixture was directly chromatographed on a column of 4.5 kg of silica gel, eluting with 9 :1 (v/v) ethyl acetate :acetone to give 20.4 g of the desired product, m.p. 218-220°C.

Continued elution with acetone gave 5.2 g of the methanol addition product, S-(2-pyridyl)-1α-methoxy-4-methyl-3-oxo-4-aza-5α-androstane-17β-thiocarboxylate, m.p. 221-223°C as a by-product.

EXAMPLE 2

Proceeding in a similar fashion to that of Example 1 Step C, the product of Step A Example 1 was converted into S-(2-pyridyl) 3-oxo-4-aza-5α-androst-1-ene-17β-thiocarboxylate, m.p. 230-232°C.

EXAMPLE 3

22-Methyl-4-aza-21-nor-5α-chol-1-ene-3,20 dione (first method)

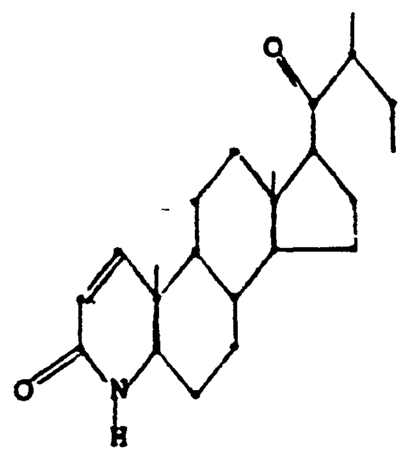

To a solution of 7.2 g of the product of Example 2, viz. S-(2-pyridyl) 3-oxo-4-aza-5α-androst-1-ene-17β-thiocarboxylate, in 288 ml of tetrahydrofuran was added at −78°C 33.6 ml of 1.3M sec-butylmagnesium chloride. After 30 minutes at −78°C the solution was allowed to reach room temperature and was then treated with saturated aqueous NaCl solution. The product was extracted into dichloromethane and was washed with saturated aqueous NaCl solution into 10% aqueous NaOH solution, then dried and concentrated. The residue was eluted through 430 g of silica gel with 9 : 1 (v/v) dichloromethane-acetone to give 4.5 g of the product, m.p. 246-249°C.

EXAMPLE 4

When the procedure of Example 3 is repeated using S-(2-pyridyl) 4-methyl-3-oxo-4-aza-5α-androst-1-ene-17β-thiocarboxylate and sec-butyl magnesium chloride as the reagents, the product obtained is 4,22-dimethyl-4-aza-21-nor-5α-chol-1-en-3-one, m.p. 134-136°C.

EXAMPLE 5

22-Methyl-4-aza-21-nor-5α-chol-1-ene-3,20-dione (second method)

A solution of 21 g of 22-methyl-4-aza-21-nor-5α-cholane-3,20-dione and 29.49 g of benzeneseleninic anhydride in 552 ml of chlorobenzene was refluxed with water separation for 4 hours. The mixture was concentrated and the residue was redissolved in dichloromethane. After washing with 10% (w/v) aqueous sodium hydroxide, 10% (w/v) hydrochloric acid and saturated aqueous sodium hydroxide, the solution was dried and concentrated to 45 g of yellow residue. This was chromatographed on 1.5 kg of silica gel packed in dichloromethane and eluted with ethyl acetate to give 10.6 g of the product, m.p. 248-251°C.

EXAMPLE 6

Step A

The procedure of Example 3 is followed using S-(2-pyridyl) 3-oxo-4-aza-5α-androstene-17β-thiocarboxylate and isobutyl magnesium chloride as reagents. The product is 23-methyl-4-aza-21-nor-5α-cholane-3,20-dione, m.p. 220-222°C.

Step B

When the procedure of Example 5 is repeated using 23-methyl-4-aza-21-nor-5α-cholane-3,20-dione, which can be obtained as described in Step A, as starting material, the product obtained is 23-methyl-4-aza-21-nor-5α-chol-1-ene-3,20-dione, m.p. 283-296°C.

## Claims

### Claims for the following Contracting States : BE, CH, DE, FR, GB, IT, LI, LU, NL, SE

1. A compound of the formula :

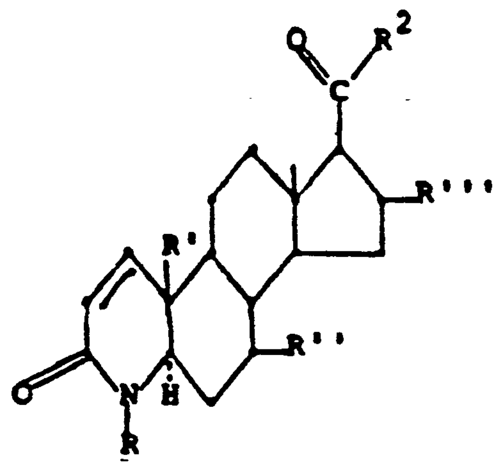

IA

in which R is hydrogen, methyl or ethyl ;
$R^2$ is a branched alkyl radical having up to 12 carbon atoms ;
R′ is hydrogen or methyl ;
R″ is hydrogen, α-methyl or β-methyl, and
R‴ is hydrogen, α-methyl or β-methyl.

2. A compound as claimed in Claim 1 having the formula :

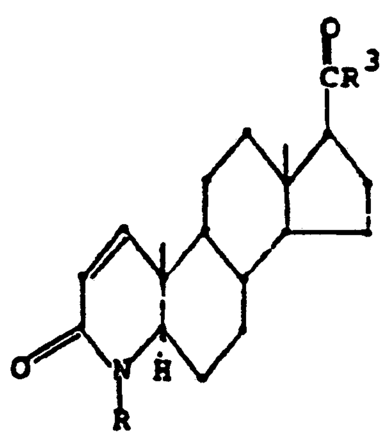

in which R is hydrogen, methyl or ethyl and $R^3$ is branched chain alkyl of from 4-8 carbon atoms.

3. 22-Methyl-4-aza-21-nor-5α-chol-1-ene-3,20-dione.

4. 23-Methyl-4-aza-21-nor-5α-chol-1-ene-3,20-dione.

5. 4,22-Dimethyl-4-aza-21-nor-5α-chol-1-en-3-one.

6. A compound as claimed in any one of Claims 1 to 5 for use in treating one or more of the hyperandrogenic

conditions of acne vulgaris, seborrhea, female hirsutism, and benign prostatic hypertrophy by oral, parenteral or topical administration.

7. A pharmaceutical composition comprising a pharmaceutically acceptable carrier and a compound as claimed in any one of Claims 1 to 5.

**Claims for the following Contracting State : AT**

1. A method of preparing a compound of the formula :

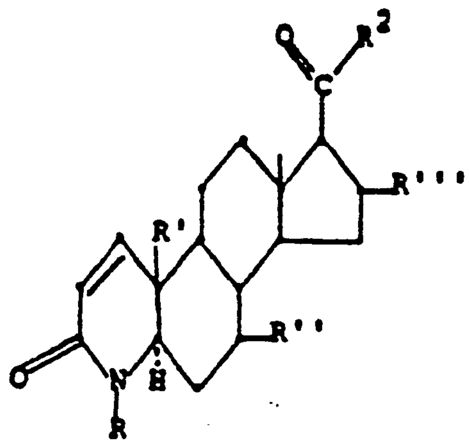

IA

in which R is hydrogen, methyl or ethyl ;
$R^2$ is a branched alkyl radical having up to 12 carbon atoms ;
R′ is hydrogen or methyl ;
R″ is hydrogen, α-methyl or β-methyl, and
R‴ is hydrogen, α-methyl or β-methyl,
that comprises reacting a compound of formula :

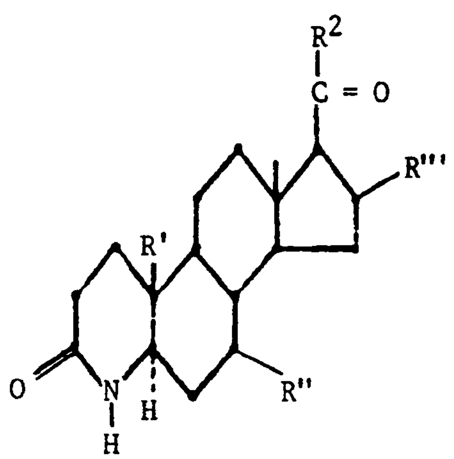

XIA

in which $R^2$, R′, R″ and R‴ are as desired in the product with a dehydrogenating agent to introduce a 1(2)-double bond, and if necessary N-methylating or N-ethyating the compound so produced.

2. A method for preparing a compound having the Formula I in Claim 1 in which R′ is methyl or ethyl, comprising refluxing a 17β-carboxy-4-(methyl or ethyl)-4-aza-5α-androst-1-en-3-one of formula :

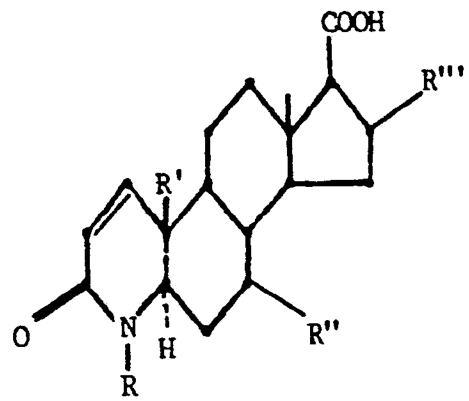

VI

in which R, R', R" and R'" are as defined in Claim 1, with triphenyl phosphine and 2,2'-dipyridyl disulphide in an inert solvent to produce a 2-pyridylthio ester and reacting the latter with an $R^2$ magnesium chloride or bromide or an $R^2$ lithium compound.

3. A method as claimed in Claim 1 as applied to the preparation of a compound having the formula :

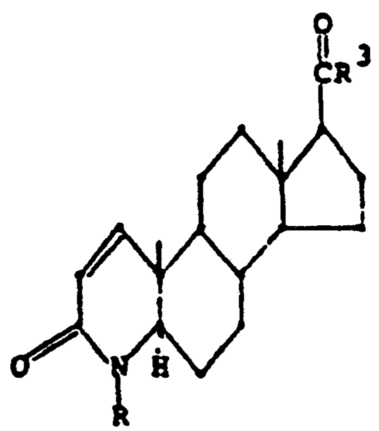

in which R is hydrogen, methyl or ethyl and $R^3$ is branched chain alkyl of from 4-8 carbon atoms.

4. A method as claimed in Claim 2 as applied to the preparation of a compound having the formula set forth in Claim 3, but in which R is methyl or ethyl.

5. A method as claimed in Claim 1 or 3 as applied to the preparation of 4,22-dimethyl-4-aza-21-nor-5α-chol-1-en-3-one.

6. A method as claimed in Claim 2 or 4 as applied to the preparation of 23-methyl-4-aza-21-nor-5α-chol-1-ene-3,20-dione.

7. A method as claimed in Claim 2 or 4 as applied to the preparation of 22-methyl-4-aza-21-nor-5α-chol-1-ene-3,20-dione.

8. A compound obtained by a method as claimed in any one of Claims 1 to 7 for use in treating one or more of the hyperandrogenic conditions of acne vulgaris, seborrhea, female hirsutism, and benign prostatic hypertrophy by oral, parenteral or topical administration.

9. A pharmaceutical composition comprising a pharmaceutically acceptable carrier and a compound obtained by a method as claimed in a any one of Claims 1 to 7.

**Patentansprüche**

**Patentansprüche für folgende Vertragsstaaten : BE, CH, DE, FR, GB, IT, LI, LU, NL, SE**

1. Verbindung der Formel :

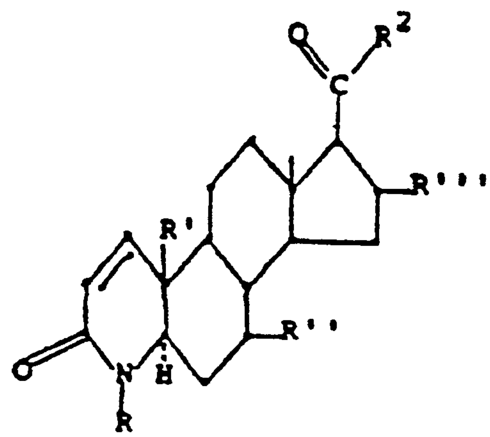

IA

worin R Wasserstoff, Methyl oder Ethyl ist ;
$R^2$ ein verzweigter Alkylrest mit bis zu 12 Kohlenstoffatomen ist ;
R' Wasserstoff oder Methyl ist ;
R" Wasserstoff, α-Methyl oder β-Methyl ist und
R''' Wasserstoff, α-Methyl oder β-Methyl ist.

2. Verbindung nach Anspruch 1 der Formel :

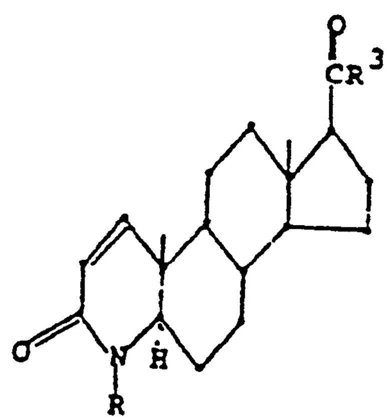

worin R Wasserstoff, Methyl oder Ethyl ist und $R^3$ verzweigtkettiges Alkyl mit 4 bis 8 Kohlenstoffatomen ist.

3. 22-Methyl-4-aza-21-nor-5α-chol-1-en-3,20-dion.

4. 23-Methyl-4-aza-21-nor-5α-chol-1-en-3,20-dion.

5. 4,22-Dimethyl-4-aza-21-nor-5α-chol-1-en-3-on.

6. Verbindung nach einem der Ansprüche 1 bis 5 zur Verwendung bei der Behandlung eines oder mehrerer hyperandrogener Zustände von Akne vulgaris, Seborrhö, Hirsutismus der Frau und gutartiger Hypertrophie der Prostata durch orale, parenterale oder topische Verabreichung.

7. Pharmazeutische Zusammensetzung, welche einen pharmazeutisch annehmbaren Träger und eine Verbindung nach einem der Ansprüche 1 bis 5 umfaßt.

**Patentansprüche für folgenden Vertragsstaat : AT**

1. Verfahren zur Herstellung einer Verbindung der Formel :

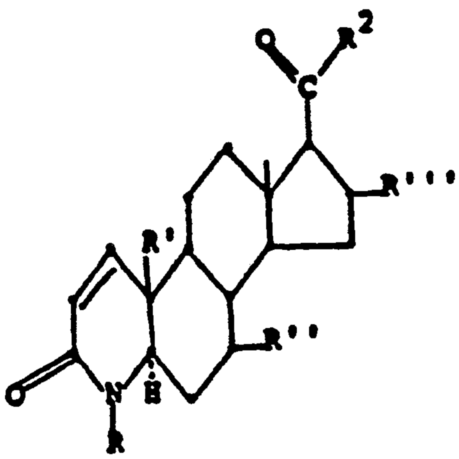

IA ,

worin R für Wasserstoff, Methyl oder Ethyl steht ;
$R^2$ ein verzweigter Alkylrest mit bis zu 12 Kohlenstoffatomen ist ;
R' Wasserstoff oder Methyl bedeutet ;
R" Wasserstoff, α-Methyl oder β-Methyl darstellt, und
R''' Wasserstoff, α-Methyl oder β-Methyl ist,
welches das Umsetzen einer Verbindung der Formel :

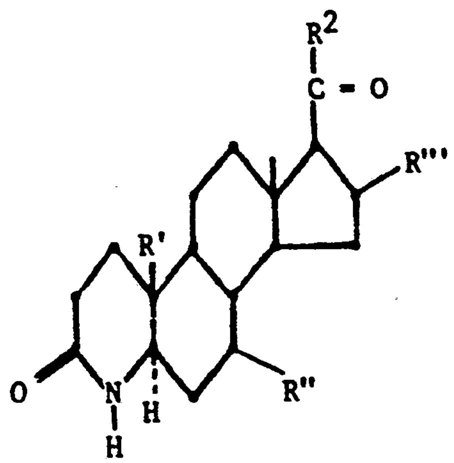

XIA ,

worin $R^2$, R', R" und R''' so sind, wie sie im Produkt gewünscht werden, mit einem Dehydrierungsmittel, um eine 1(2)-Doppelbindung einzuführen, und, wenn notwendig, N-Methylieren oder N-Ethylieren der so hergestellten Verbindung umfaßt.

2. Verfahren zur Herstellung einer Verbindung mit der Formel I aus Anspruch 1, worin R' Methyl oder Ethyl bedeutet, welches das Erhitzen unter Rückfluß von einem 17β-Carboxy-4-(methyl oder ethyl)-4-aza-5α-androst-1-en-3-on der Formel :

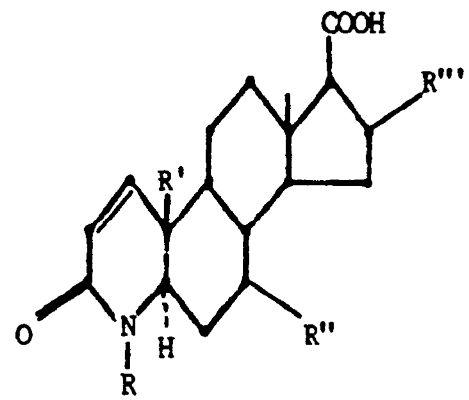

VI,

worin R, R′, R″ und R‴ wie in Anspruch 1 definiert sind, mit Triphenylphosphin und 2,2′-Dipyridyldisulfid in einem inerten Lösungsmittel, um einen 2-Pyridylthioester herzustellen, und das Umsetzen des letztgenannten mit einem $R^2$-Magnesiumchlorid oder -bromid oder einer $R^2$-Lithiumverbindung umfaßt.

3. Verfahren nach Anspruch 1, angewandt auf die Herstellung einer Verbindung mit der Formel :

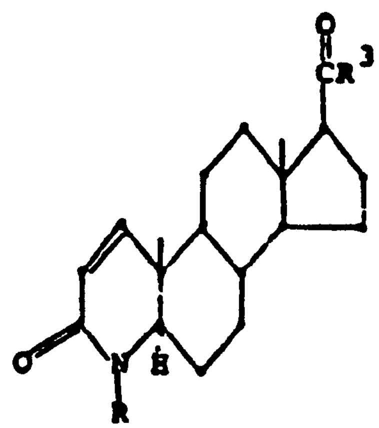

,

worin R für Wasserstoff, Methyl oder Ethyl steht und $R^3$ ein verzweigtkettiges Alkyl mit 4 bis 8 Kohlenstoffatomen darstellt.

4. Verfahren nach Anspruch 2, angewandt auf die Herstellung einer Verbindung, welche die in Anspruch 3 angegebene Formel besitzt, in welcher R jedoch für Methyl oder Ethyl steht.

5. Verfahren nach Anspruch 1 oder 3, angewandt auf die Herstellung von 4,22-Dimethyl-4-aza-21-nor-5α-chol-1-en-3-on.

6. Verfahren nach Anspruch 2 oder 4, angewandt auf die Herstellung von 23-Methyl-4-aza-21-nor-5α-chol-1-en-3,20-dion.

7. Verfahren nach Anspruch 2 oder 4, angewandt auf die Herstellung von 22-Methyl-4-aza-21-nor-5α-chol-1-en-3,20-dion.

8. Verbindung, welche durch ein Verfahren nach einem der Ansprüche 1 bis 7 erhalten wird, zur Verwendung in der Behandlung von einem oder mehreren hyperandrogenen Zuständen von Acne vulgaris, Seborrhoe, weiblichem Hirsutismus und gutartiger Prostatahypertrophie durch orale, parenterale oder topische Verabreichung.

9. Pharmazeutische Zusammensetzung, umfassend einen pharmazeutisch annehmbaren Träger und eine Verbindung, welche durch ein Verfahren nach einem der Ansprüche 1 bis 7 erhalten wird.

**Revendications**

**Revendications pour les Etats contractants suivants : BE, CH, DE, FR, GB, IT, LI, LU, NL, SE**

1. Composé de formule

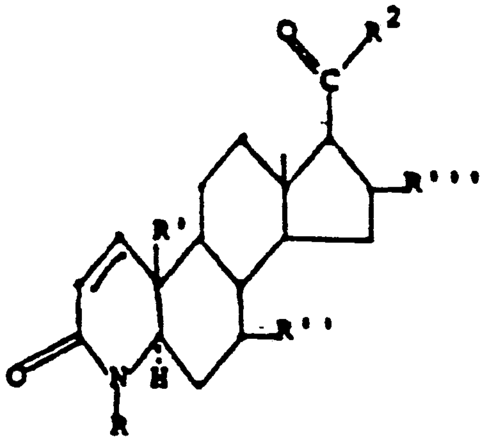

IA

dans laquelle R représente un atome d'hydrogène, un groupe méthyle ou éthyle ;
$R^2$ représente un radical alkyle ramifié ayant jusqu'à 12 atomes de carbone ;
R' représente un atome d'hydrogène ou le groupe méthyle ;
R" représente un atome d'hydrogène, un groupe α -méthyle ou β-méthyle et ;
R'" représente un atome d'hydrogène, un groupe α -méthyle ou β-méthyle.

2. Composé selon la revendication 1, répondant à la formule :

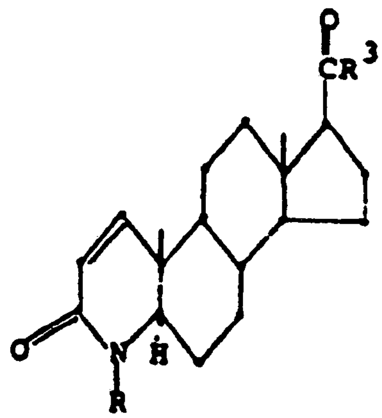

dans laquelle R représente un atome d'hydrogène, un groupe méthyle ou éthyle et $R^3$ est un groupe alkyle à chaîne ramifiée avec de 4 à 8 atomes de carbone.

3. 22-méthyl-4-aza-21-nor-5α-chol-1-ène-3,20-dione.

4. 23-méthyl-4-aza-21-nor-5α-chol-1-ène-3,20-dione.

5. 4,22-diméthyl-4-aza-21-nor-5α-chol-1-èn-3-one.

6. Composé selon l'une quelconque des revendications 1 à 5, à utiliser dans le traitement d'un ou plusieurs parmi les états d'hyperandrogénie d'acné vulgaire, de séborrhée, d'hirsutisme féminin, et d'hypertrophie prostatique bénigne, par administration orale, parentérale ou topique.

7. Composition pharmaceutique, comprenant un véhicule pharmaceutiquement acceptable et un composé selon l'une quelconque des revendications 1 à 5.

**Revendications pour l'état contractant suivant : AT**

1. Procédé de préparation d'un composé de formule :

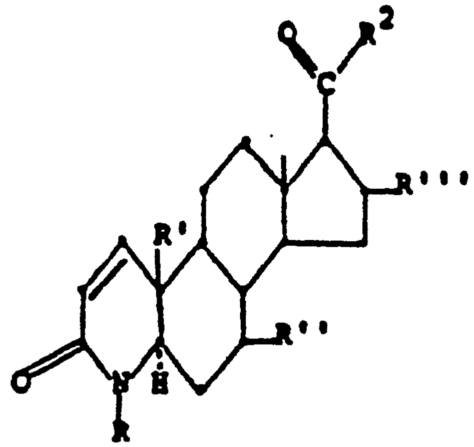

IA

dans laquelle R représente un atome d'hydrogène, le groupe méthyle ou éthyle ;
$R^2$ est un radical alkyle ramifié ayant jusqu'à 12 atomes de carbone ;
R' représente un atome d'hydrogène ou le groupe méthyle ;
R" représente un atome d'hydrogène, un groupe α-méthyle ou β-méthyle et ;
R'" représente un atome d'hydrogène, un groupe α-méthyle ou β-méthyle,
qui comprend la réaction d'un composé de formule

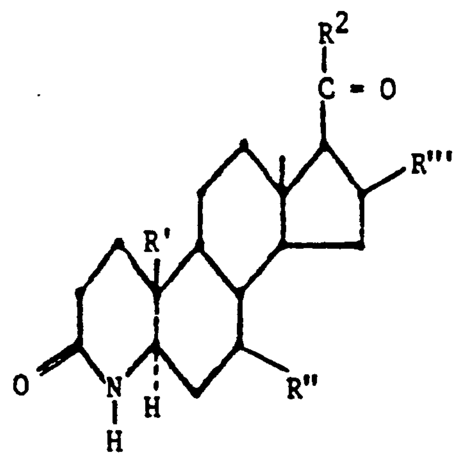

XIA

dans laquelle $R^2$, R', R" et R'" sont tels que souhaités dans le produit, avec un agent de déshydrogénation pour introduire une double liaison 1,2, et si nécessaire la N-méthylation ou la N-éthylation du composé ainsi produit.

2. Procédé de préparation d'un composé répondant à la formule I dans la revendication 1, dans laquelle R' est le groupe méthyle ou éthyle, qui comprend le chauffage au reflux d'une 17β-carboxy-4-(méthyl ou éthyl)-4-aza-5α -androst-1-ène-3-one de formule :

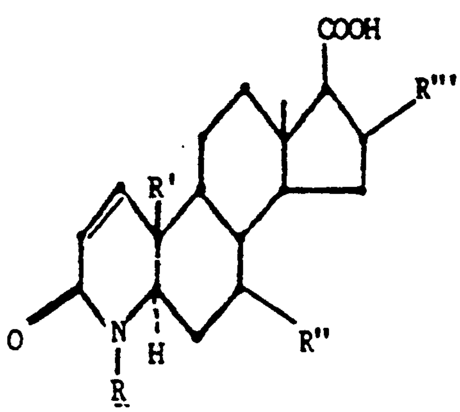

VI

dans laquelle R, R', R" et R"' sont tels que définis dans la revendication 1, avec la triphénylphosphine et le 2, 2'-dipyridyldisulfure dans un solvant inerte pour produire un ester 2-pyridylthio et la réaction de ce dernier avec un chlorure ou bromure de $R^2$ magnésium ou un composé de $R^2$-lithium.

3. Procédé selon la revendication 1, appliqué à la préparation d'un composé répondant à la formule :

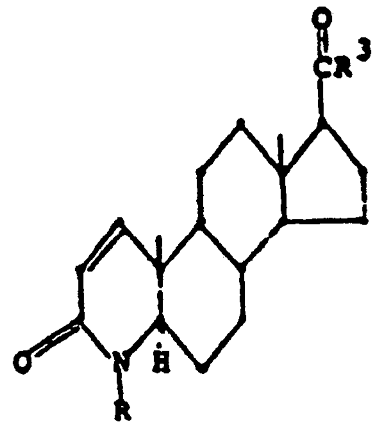

dans laquelle R est un atome d'hydrogène, un groupe méthyle ou éthyle et $R^3$ est un groupe alkyle à chaîne ramifiée avec de 4 à 8 atomes de carbone.

4. Procédé selon la revendication 2,appliqué à la préparation d'un composé répondant à la formule mentionnée dans la revendication 3, mais dans lequel R est le groupe méthyle ou éthyle.

5. Procédé selon la revendication 1 ou 3, appliqué à la préparation de la 4,22-diméthyl-4-aza-21-nor-5α-chol-1-èn-3-one.

6. Procédé selon la revendication 2 ou 4, appliqué à la préparation de la 23-méthyl-4-aza-21-nor-5α-chol-1-ène-3,20-dione.

7. Procédé selon la revendication 2 ou 4, appliqué à la préparation de la 22-méthyl-4-aza-21-nor-5α-chol-1-ène-3,20-dione.

8. Composé obtenu par un procédé selon l'une quelconque des revendications 1 à 7, à utiliser dans le traitement d'un ou plusieurs parmi les états d'hyperandrogénie d'acné vulgaire, de séborrhée, d'hirsutisme féminin et d'hypertrophie prostatique bénigne, par administration orale, parentérale ou topique.

9. Composition pharmaceutique qui comprend un véhicule pharmaceutiquement acceptable et un composé obtenu par un procédé selon l'une quelconque des revendications 1 à 7.